# EUROPEAN PATENT SPECIFICATION

(11) **EP 3 517 107 B1**
(45) Date of publication and mention of the grant of the patent: **01.11.2023**
(21) Application number: 17853177.8
(22) Date of filing: 22.09.2017
(51) Int. Cl.: A61K 31/09, A61P 15/00, A23L 33/10, A23L 33/105

(54) **AGENT FOR PREVENTING OR AMELIORATING REDUCTION IN OVARIAN FUNCTION**
MITTEL ZUR VERHINDERUNG ODER VERBESSERUNG DER REDUZIERUNG DER EIERSTOCKFUNKTION
AGENT DE PRÉVENTION OU D'AMÉLIORATION DE LA RÉDUCTION DE LA FONCTION OVARIENNE

(30) Priority: 26.09.2016 JP 2016187572
(43) Date of publication of application: 31.07.2019
(73) Proprietor: St. Marianna University School of Medicine, Kawasaki-shi Kanagawa 216-8511 (JP); Partners Co., Ltd., Yokohama-shi, Kanagawa 222-0033 (JP); Okamoto, Naoki, Kawasaki-shi, Kanagawa 214-0036 (JP)
(72) Inventor: OKAMOTO, Naoki, Kawasaki-shi Kanagawa 214-0036 (JP); KAWAMURA, Kazuhiro, Kawasaki-shi Kanagawa 216-8511 (JP); HOSOKAWA, Tadahiro, Yokohama-shi Kanagawa 222-0033 (JP)
(74) Representative: De Clercq & Partners
(86) International application number: PCT/JP2017/034361
(87) International publication number: WO 2018/056409

(56) References cited:
- WO-A1-2014/010685
- CN-A- 103 565 782
- JP-A- 2014 520 526
- US-A1- 2009 175 803
- US-A1- 2013 217 782
- Haoru Zhang ET AL: "Effects of pterostilbene on treating hyperprolactinemia and related mechanisms", Am J Transl Res, 30 July 2016 (2016-07-30), XP055690451, Retrieved from the Internet: URL:https://www.ncbi.nlm.nih.gov/pmc/artic les/PMC4969441/pdf/ajtr0008-3049.pdf [retrieved on 2020-04-29]
- Gina Lowe ET AL: "Abstract 3215: Role of pterostilbene on proliferation, invasion, and migration in ovarian and uterine cancer cells | Cancer Research", Cancer Research, 1 October 2014 (2014-10-01), XP055690802, Retrieved from the Internet: URL:https://cancerres.aacrjournals.org/con tent/74/19_Supplement/3215 [retrieved on 2020-04-30]
- CHENG Y. et al .,: "SIRTl activation by pteroslilbene attenuates the skeletal muscle oxidative stress injury and mitochondrial dysfunction induced by ischemia r e perfusion injury.", Apoptosis, vol. 21, no. 08, August 2016 (2016-08), pages 905-916, XP035991303,
- BELVIRANLI M.: "Chapter 5: Well- Known Antioxidants and Newcomers in Sport Nutrition Coenzyme QlO Quercetin, Resveratrol, Pterostilbene, Pycnogenol, and Astaxanthin .", Antioxidants in Sports Nutrition, 2015, pages 79-102, XP009515593, ISBN: 9781466567573
- Naoki OKAMOTO: "0-88 Resveratrol Sesshu ni yoru Aging Ranshi no Shitsu no Kaizen - Ransonai Sirtuin Hatsugen no Henka", Journal of mammalian ova research, vol. 32, no. 2, April 2015 (2015-04), page s-54,
- Naoki OKAMOTO: "0-222 Resveratrol Sesshu ni yoru Aging Ranshi no Shitsu no Kaizen - Ransonai Sirtuin Hatsugen no Henka", Journal of Japan society for Reproductive Medicine, vol. 59, no. 4, 2014, page 216, XP009516428,
- Takeo s.: "Resveratrol improves the mitochondrial function and fertilization outcome of bovine oocytes", Journal of Reproduction and Developrent, vol. 60, no. 2, 2014, pages 92-99, XP055586256,
- ITAMI N.: "Resveratrol improves the quality of pig oocytes derived fro~ early antral !ollicles through sirtuin 1 activation .", Theriogenology, vol. 83, no. 8, 2015, pages 1360-1367, XP055586260,

## Description

### TECHNICAL FIELD

The present invention relates to an agent for preventing or improving deterioration of ovarian function, the agent comprising pterostilbene.

### BACKGROUND ART

In females, there is a close association between age and pregnancy, and the pregnancy rate tends to decrease as the age increases. Major reasons for this fact include a decrease in the number of, and deterioration of the quality of, eggs in the ovaries. Females already have the source of all eggs in the ovaries at birth, and the number and the quality of the eggs decrease as the age increases. Nowadays, the age at marriage increases year by year, and it can therefore be said that an increasing number of females, even without an evident cause of infertility, have difficulty in becoming pregnant due to deterioration of ovarian function caused by aging, that is, due to a decrease in the number of, and deterioration of the quality of, the eggs in the ovaries.

In recent years, techniques in the assisted reproductive technology such as in vitro fertilization and microinsemination have advanced, but treatment of a decrease in the pregnancy rate due to deterioration of ovarian function is in fact still impossible even with the cutting-edge techniques in reproductive medicine. Thus, means for suppressing the deterioration of ovarian function has been demanded.

Pterostilbene is a compound having a stilbene skeleton, contained in, for example, the legme Indian kino tree. Pterostilbene is known to have physiological activities such as antioxidant action, prophylactic/therapeutic action on thrombosis (Patent Document 1), reduction of LDL cholesterol, improvement of insulin resistance, anti-inflammatory action, and cancer-suppressing action (Non-patent Document 1).

### PRIOR ART DOCUMENTS

### [Patent Document]

[Patent Document 1] JP 2016-44134 A

### [Non-patent Document]

[Non-patent Document 1] Alternative Medicine Review 2010; 15: 159-163
Takeo S.: Journal of Reproduction and Developrent, vol. 60, no. 2, 2014, pages 92-99; discloses that resveratrol improves the mitochondrial function and fertilization outcome of oocytes.
ITAMI N.: Theriogenology, vol. 83, no. 8, 2015, pages 1360-1367 discloses that resveratrol improves the quality of pig oocytes.

### SUMMARY OF THE INVENTION

### TECHNICAL PROBLEM

In today's society, mismatches between the suitable ages for pregnancy and the ages at which people desire pregnancy have led to an increase in the number of cases of modern-type infertility, which is caused by deteriorated ovarian function at the ages at which people desire pregnancy. Therefore, means for prevention and/or improvement of such infertility has been demanded. Also in the fields of animal husbandry and the like, means for suppressing a decrease in the pregnancy rate due to deteriorated ovarian function has been demanded. An object of the present invention is to provide a preparation effective for prevention and/or improvement of deterioration of ovarian function in order to satisfy these demands.

### SOLUTION TO PROBLEM

In order to solve the above problems, the present inventors intensively studied. As a result, the present inventors discovered that pterostilbene is effective for prevention and/or improvement of deterioration of ovarian function caused by aging or the like. Based on this discovery, the present inventors completed the present invention.

That is, the present invention is as follows.
[1] An agent for preventing or improving deterioration of ovarian function, the agent comprising pterostilbene, wherein the reduction of an implantation rate of fertilized eggs is suppressed, or the implantation rate of fertilized eggs is increased; wherein the reduction of a live offspring rate is suppressed, or the live offspring rate is increased; wherein the abortion rate is reduced; or wherein the deterioration of ability of reproduction is improved.
[2] A pharmaceutical composition for preventing or improving deterioration of ovarian function, the composition comprising the agent for preventing or improving deterioration of ovarian function according to [1],
[3] A food or beverage composition for preventing or improving deterioration of ovarian function, the composition comprising the agent for preventing or improving deterioration of ovarian function according to [1],

### ADVANTAGEOUS EFFECTS OF THE INVENTION

According to the present invention, a preparation effective for prevention and/or treatment of deterioration of ovarian function caused by aging as defined in the claims is provided.

### BRIEF DESCRIPTION OF THE DRAWINGS

[FIG. 1] FIG. 1 is a diagram showing an experimental design using pterostilbene.
[FIG. 2] FIG. 2 is a diagram showing the mouse body weights in each group at the beginning and at the end of a pterostilbene ingestion test. A of FIG. 2 shows the body weights of each group of mice at 25 weeks old (at the beginning of the test). B of FIG. 2 shows the body weights of each group of mice at 47 weeks old (at the end of the test).
[FIG. 3] FIG. 3 is a diagram showing the results of in vitro fertilization and embryo culture of mice that ingested pterostilbene. A of FIG. 3 shows the numbers of ovulated eggs of each group of mice. B of FIG. 3 shows the fertility rates of each group of mice. C of FIG. 3 shows the blastocyst formation rates of each group of mice.
[FIG. 4] FIG. 4 is a diagram showing the results of embryo transfer of embryos obtained from mice that ingested pterostilbene. A of FIG. 4 shows the implantation rates of each group of mice. B of FIG. 4 shows the live offspring acquisition rates of each group of mice. C of FIG. 4 shows the abortion rates of each group of mice. D of FIG. 4 shows visual observation (a photograph) of mice born after embryo transfer of embryos obtained from mice that ingested pterostilbene for 22 weeks, and placentas.
[FIG. 5] FIG. 5 is a diagram showing the body weights of live offspring obtained from mice that ingested pterostilbene, and the placental weights. A of FIG. 5 shows the body weights of live offspring of each group of mice. B of FIG. 5 shows the placental weights of each group of mice.
[FIG. 6] FIG. 6 is a diagram (photograph) showing an offspring obtained from a mouse in a group in which pterostilbene was ingested for 22 weeks, and offspring of this offspring.
[FIG. 7] FIG. 7 is a diagram showing an experimental design using resveratrol.
[FIG. 8] FIG. 8 is a diagram showing the mouse body weights in each group at the beginning and at the end of a resveratrol ingestion test. A of FIG. 8 shows the body weights of each group of mice at 25 weeks old (at the beginning of the test). B of FIG. 8 shows the body weights of each group of mice at 47 weeks old (at the end of the test).
[FIG. 9] FIG. 9 is a diagram showing the results of in vitro fertilization and embryo culture of mice that ingested resveratrol. A of FIG. 9 shows the numbers of ovulated eggs of each group of mice. B of FIG. 9 shows the fertility rates of each group of mice. C of FIG. 9 shows the blastocyst formation rates of each group of mice.
[FIG. 10] FIG. 10 is a diagram showing the results of embryo transfer of embryos obtained from mice that ingested resveratrol. A of FIG. 10 shows the implantation rates of each group of mice. B of FIG. 10 shows the live offspring acquisition rates of each group of mice. C of FIG. 10 shows the abortion rates of each group of mice.
[FIG. 11] FIG. 11 is a diagram showing an experimental design using pterostilbene.
[FIG. 12] FIG. 12 is a diagram showing the numbers of ovulated eggs in mice that ingested pterostilbene.
[FIG. 13] FIG. 13 is a diagram showing the results of analysis of mitochondrial activity in eggs of mice that ingested pterostilbene. A of FIG. 13 shows the result of observation with a confocal laser microscope (photograph). B of FIG. 13 shows the average intensity of mitochondrial activity in the eggs.
[FIG. 14] FIG. 14 is a diagram showing the ATP amounts in eggs of mice that ingested pterostilbene.
[FIG. 15] FIG. 15 is a diagram showing the copy numbers of mitochondrial DNA in eggs of mice that ingested pterostilbene.
[FIG. 16] FIG. 16 is a diagram showing the serum pterostilbene or resveratrol concentrations in mice that ingested pterostilbene or resveratrol. A of FIG. 16 shows the serum pterostilbene concentrations in the mice that ingested pterostilbene. B of FIG. 16 shows the serum resveratrol concentrations in the mice that ingested resveratrol.
[FIG. 17] FIG. 17 is a diagram showing the serum pterostilbene or resveratrol concentrations in mice that ingested the respective compounds.
[FIG. 18] FIG. 18 is a diagram (photographs) showing TUNEL staining images of an ovarian tissue of a mouse that ingested pterostilbene. A of FIG. 18 shows a TUNEL staining image of the mouse ovarian tissue. B of FIG. 18 shows a magnified image of A of FIG. 18.
[FIG. 19] FIG. 19 is a diagram (photographs) showing 8-OhdG staining images of an ovarian tissue of a mouse that ingested pterostilbene.

### DESCRIPTION OF THE EMBODIMENTS

One mode of the present invention relates to an agent for preventing or improving deterioration of ovarian function, the agent comprising pterostilbene. The pterostilbene used in the present invention (4'-hydroxy-3,5-dimethoxy-trans-stilbene) (trans-1-(3,5-dimethoxyphenyl)-2-(4-hydroxyphenyl)ethylene) is a compound represented by the chemical formula C₁₆H₁₆O₃.

### (Pterostilbene)

The agent for preventing or improving deterioration of ovarian function of the present invention is characterized in that it contains pterostilbene as an effective component. The pterostilbene used in the present invention may be either chemically synthesized or derived from a naturally occurring product. Examples of the pterostilbene derived from a naturally occurring product include those obtained from Indian kino tree (*Pterocarpus marsupium*), which is a plant belonging to the genus *Pterocarpus* of the family Fabaceae. Examples of the production method for pterostilbene include a method in which, after crushing a whole Indian kino tree including stem, branch, leaf, flower, and/or root, or crushing stem, branch, leaf, flower, and/or root by a conventional method, a pterostilbene-containing extract is obtained from the resulting crushed powder by a conventional method. In the present invention, the pterostilbene may be in the state of an extract or a crushed product, or may be a product purified as appropriate. In the present invention, one of, or a combination of two or more of, such extracts, crushed powders, purified products, and chemically synthesized products containing pterostilbene may be used. The pterostilbene may also be a commercially available product.

### (Agent for Preventing or Improving Deterioration of Ovarian Function)

The agent for preventing or improving deterioration of ovarian function of the present invention has an action for prevention of deterioration of ovarian function, and for improvement/treatment of deteriorated ovarian function. The action for prevention or improvement/treatment of deterioration of ovarian function includes suppression of reduction of the implantation rate of fertilized eggs, or increasing of the implantation rate of fertilized eggs; suppression of reduction of the live offspring rate, or increasing of the live offspring rate; suppression of increasing of the abortion rate, or reduction of the abortion rate.

The agent for preventing or improving deterioration of ovarian function of the present invention has an action for prevention of deterioration of ovarian function, and for improvement/treatment of deteriorated ovarian function. Thus, the agent for preventing or improving deterioration of ovarian function of the present invention is effective for, for example, prevention, improvement, or treatment of infertility due to deterioration of ovarian function caused by aging

Examples of the subjects to which the agent for preventing or improving deterioration of ovarian function of the present invention is to be applied include, but are not limited to, mammals requiring prevention, improvement, or treatment of infertility due to deterioration of ovarian function. Examples of the mammals include, but are not limited to, human; and non-human animals such as pigs, cows, bovids, horses, and buffalos, which are animals having commercial values for production of meat and dairy production.

The method of producing the agent for preventing or improving deterioration of ovarian function of the present invention is not limited as long as the agent contains pterostilbene as an effective component. That is, the agent may be produced using a conventional technique for preparation of pharmaceuticals.

The agent for preventing or improving deterioration of ovarian function of the present invention may be provided as a pharmaceutical composition by including, when necessary, an auxiliary agent(s) conventionally used in the technical field of formulation of pharmaceuticals, such as excipients, binders, antiseptics, oxidation stabilizers, disintegrators, lubricants, and correctives, in addition to the pterostilbene as an effective component.

The content of pterostilbene in the pharmaceutical composition may be usually 0.01% by mass to 10% by mass, preferably 0.05% by mass to 5% by mass, more preferably 0.01% by mass to 0.5% by mass with respect to the total mass of the pharmaceutical composition.

The administration route of the agent for preventing or improving deterioration of ovarian function of the present invention may be either oral administration or parenteral administration. The dosage form is appropriately selected depending on the administration route. Examples of the dosage form include injection solutions, infusion solutions, powders, granules, tablets, capsules, balls, enteric coated tablets, troches, solutions for internal use, suspensions, emulsions, syrups, solutions for external use, fomentations, ointments, lotions, suppositories, and enteral nutrients.

The dose of the agent for preventing or improving deterioration of ovarian function of the present invention varies depending on the purpose of administration (for example, symptoms and severity of the symptoms) and conditions (for example, the age and the body weight) of the subject to whom the agent is to be administered. The agent may be administered at a dose of usually 0.1 mg to 1000 mg, preferably 1 mg to 100 mg per day in terms of the amount of pterostilbene for oral administration to an adult.

The dosing period varies depending on the purpose of administration (for example, symptoms and severity of the symptoms) and conditions (for example, the age and the body weight) of the subject to whom the agent is to be administered. The dosing period may be 1 day to 200 days, preferably 7 days to 100 days. The agent for preventing or improving deterioration of ovarian function of the present invention is highly safe for living bodies, and may be administered for a long period exceeding the above-described period depending on the degree of improvement of symptoms, or for the purpose of preventing development or recurrence of symptoms.

### (Food or Beverage Composition, and the Like)

The agent for preventing or improving deterioration of ovarian function of the present invention may be used not only as a pharmaceutical, but also as a quasi drug, functional food, food for specified health use, dietary supplement, food or beverage, or the like. In cases where the agent is used as a quasi drug, the agent may be used, when necessary, together with various auxiliary agents conventionally used in the technical field of quasi drugs, cosmetics, or the like. In cases where the agent is used as a functional food, food for specified health use, dietary supplement, or food or beverage, the agent may be used, when necessary, together with additives conventionally used for food, such as sweeteners, spices, seasonings, antiseptics, preservatives, microbicides, and antioxidants. The agent may be used in a desired form, for example, in the form of a solid, in the form of a solution, in the form of a suspension, in the form of a syrup, in the form of a granule, in the form of a cream, in the form of a paste, or in the form of a jelly. The agent may be molded, if necessary. The ratios of pterostilbene contained in these forms are not limited, and may be appropriately selected depending on the purpose of use, mode of use, and amount of use. The content of pterostilbene in the quasi drug, functional food, food for specified health use, dietary supplement, or food or beverage may be, for example, usually 0.01% by mass to 10% by mass, preferably 0.05% by mass to 5% by mass, more preferably 0.01% by mass to 0.5% by mass with respect to the total mass of the composition. The quasi drug, functional food, food for specified health use, dietary supplement, food or beverage, or the like may be produced by a conventional production method in each field.

### EXAMPLES

Details of the present invention are described below by way of Examples. However, the present invention is not limited to the following Examples.

### <Example 1>

### (Experimental Animals)

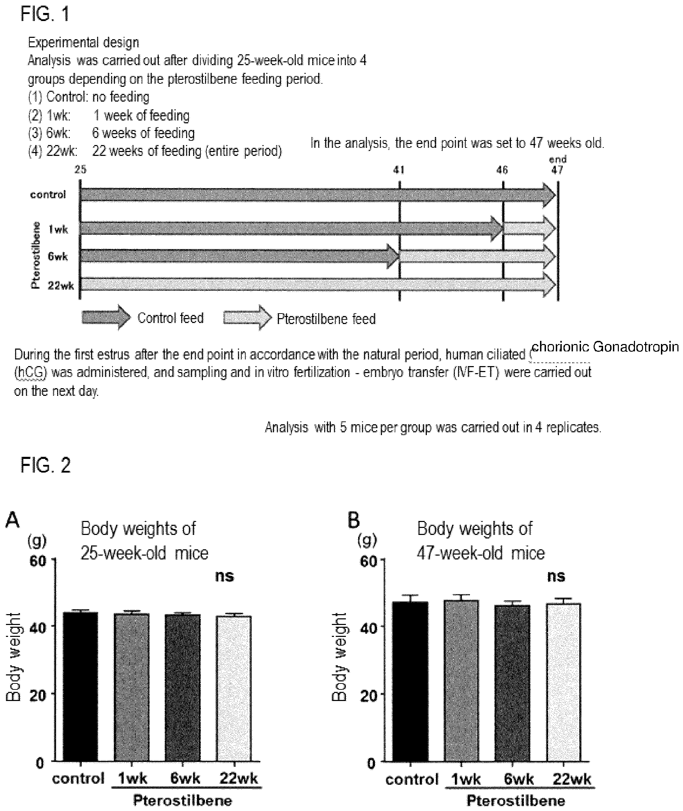

Twenty female ICR mice of 25 weeks old (CLEA Japan Inc., Tokyo, Japan) were randomly divided into 4 cages at 5 mice per cage. The mice were kept at a room temperature of 22°C at a humidity of 55% in a 12-hour light/dark cycle. Each mouse was fed with 6 g per day of mouse feed, which was MF control feed (Oriental Yeast Co., Ltd., Tokyo, Japan) or pterostilbene (90% Silvinol, Sabinsa Japan Corporation, Tokyo, Japan)-containing feed (containing 0.04% by mass pterostilbene), in the schedule shown in the experimental design in FIG. 1. The mice were provided with water ad libitum. During the feeding period, the body weight was measured once a week. The body weights (group average) of each group of mice at Week 25 and the body weights (group average) of each group of mice at Week 47 are shown in FIG. 2.

Handling and rearing of all mice were carried out according to a guideline provided by the laboratory animal facility of St. Marianna University School of Medicine.

As shown in FIG. 2, at the beginning of the test, that is, among the 25-week-old mice, no significant difference in the body weight was found between the control group and the group in which pterostilbene is to be administered for 22 weeks. Also at the end of the test, that is, among the 47-week-old mice, no significant difference in the body weight was found between the control group and the group in which pterostilbene was administered for 22 weeks.

### (In Vitro Fertilization and Embryo Culture)

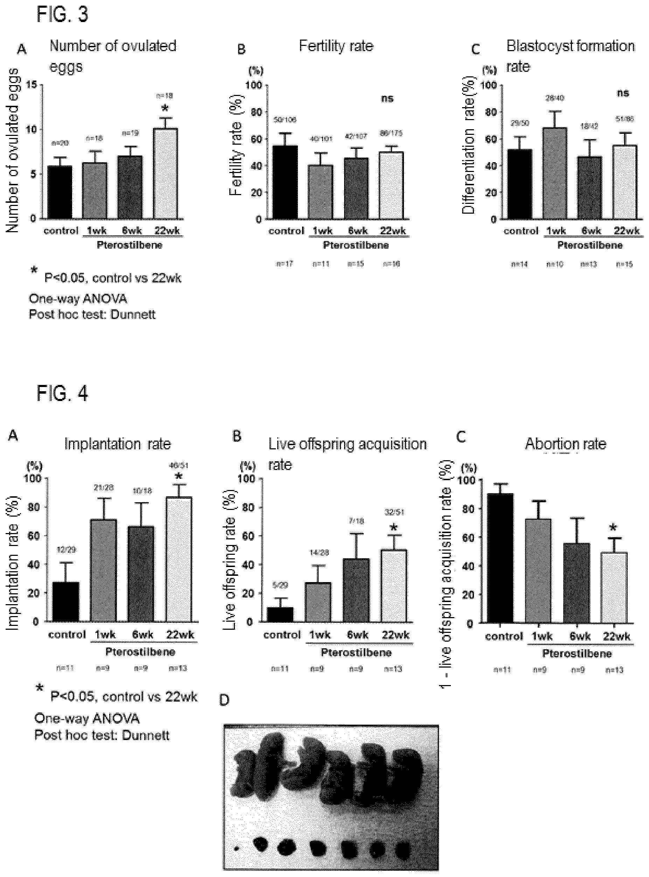

For mice that became older than 47 weeks old, the sexual cycle was investigated by the vaginal smear test. During the proestrus, 10 IU of Gonadotropin (ASKA Pharmaceutical Co., Tokyo, Japan) was intraperitoneally administered. Fifteen hours after the administration, cumulus oocyte complexes (COCs) were collected from oviduct, and then precultured for 30 minutes in 100 mL of TYH medium (LSI Medience corporation, Tokyo, Japan). During this process, male ICR mice (10 to 12 weeks old) were subjected to general anesthesia with Somnopentyl anesthetic (Kyoritsu Seiyaku Corporation, Tokyo, Japan), and cauda epididymis was removed therefrom. The cauda epididymis was partially incised, and sperms contained therein were collected by pushing out. The collected mass of sperms was immersed in 400 µL of TYH medium placed in a 1.5-mL microtube, and allowed swim-up for 10 minutes in a CO₂ incubator (37°C, 5% CO₂, 95% in air). The sperms after the swim-up were added to 100 mL of the TYH medium containing COCs to a final concentration of 2 to 3 × 10⁵/mL, and then culture was performed for 5 to 6 hours in a CO₂ incubator (37°C, 5% CO₂, 95% in air). The cultured eggs were placed in 30 mL of KSOM medium (Merck Millipore, Darmstadt, Germany), and sperms were removed therefrom while the accurate number of ovulated eggs was calculated. Subsequently, the eggs were transferred to 30 mL of fresh KSOM medium, and then culture was performed for 4 days in a CO₂ incubator. The fertility rate and the blastocyst formation rate were calculated as the number of embryos at the two-cell stage / the number of eggs in the metaphase of the second meiotic division (MII), and the number of blastocysts / the number of embryos at the two-cell stage, respectively. The results are shown in FIG. 3.

As shown in FIG. 3, a significant difference in the number of ovulated eggs was found between the control group and the group in which pterostilbene was administered for 22 weeks (P<0.05; One-way ANOVA, Post hoc test: Dunnett's method). As a result of the administration of pterostilbene for 22 weeks, an effective increase in the number of ovulated eggs was found.

### (Embryo Transfer and Cesarean Section)

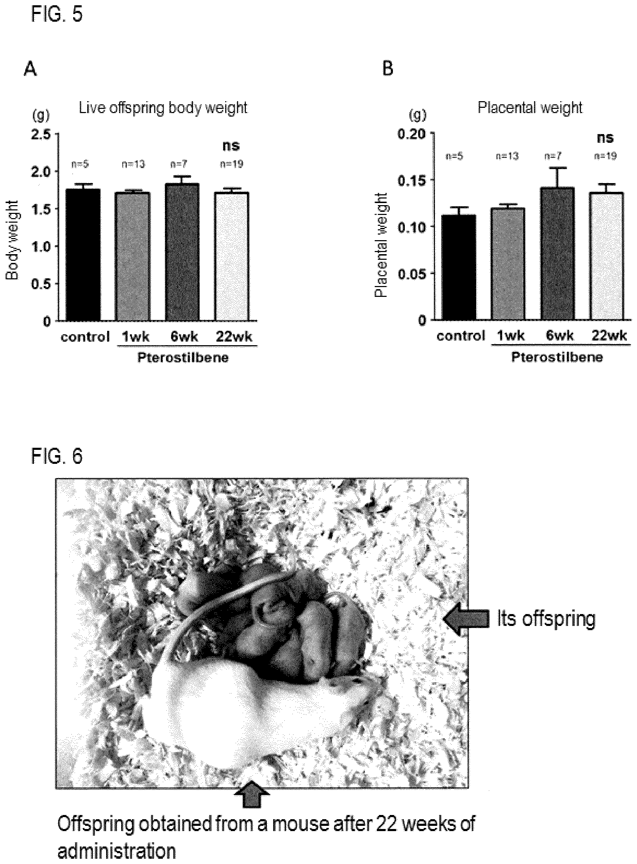

After the 4 days of culture, embryos that reached the blastocyst stage were subjected to embryo transfer. Female ICR mice of 6 to 10 weeks old were used as recipient mice. These mice were mated with male ICR mice that had been subjected to vasoligation on the day before the egg collection, and individuals for which a vaginal plug could be found on the next day were used. General anesthesia was performed with Somnopentyl anesthetic, and the uterus was exposed by incision on the back. The uterus was fixed with forceps, and the junction of oviduct was pierced with a 30-G injection needle (Dentronics, Tokyo, Japan), followed by inserting a glass capillary containing sucked blastocysts and transferring the embryos into the uterus. After the transfer, the uterus was carefully returned into the body, and the retroperitoneum and the skin were sutured. In the transfer, blastocysts obtained from each individual in each group were transferred to each recipient mouse. The day after the egg collection was defined as Day 1, and cesarean section was carried out on Day 19. Each recipient mouse was euthanized, and subjected to laparotomy, followed by removing the uterus and collecting fetuses and the placenta. The implantation rate was calculated as the number of traces of implantation / the number of embryos transferred; the live offspring acquisition rate was calculated as the number of live offspring / the number of embryos transferred; and the abortion rate was calculated as 1 - the live offspring acquisition rate. The results are shown in FIG. 4. In addition, the body weight of each live offspring and the placental weight were measured. The results are shown in FIG. 5.

As shown in FIG. 4, significant differences in the implantation rate, the live offspring acquisition rate, and the abortion rate were found between the control group and the group in which pterostilbene was administered for 22 weeks (P<0.05; One-way ANOVA, Post hoc test: Dunnett's method). The administration of pterostilbene for 22 weeks was found to be effective for increasing the implantation rate, increasing the live offspring acquisition rate, and decreasing the abortion rate. As shown in FIG. 5, no significant difference in the body weight of live offspring or the placental weight was found between the control group and the group in which pterostilbene was administered for 22 weeks.

The offspring obtained from the group in which pterostilbene was administered for 22 weeks were raised to adulthood, and subjected to a mating test. The offspring obtained from the group in which pterostilbene was administered for 22 weeks showed no remarkable abnormality, leading to normal pregnancy and delivery. Thus, these offspring were found to have normal ability of reproduction. The results are shown in FIG. 6.

### <Comparative Example 1>

An experiment was carried out using resveratrol, which is a stilbene derivative similarly to pterostilbene.

### (Experimental Animals)

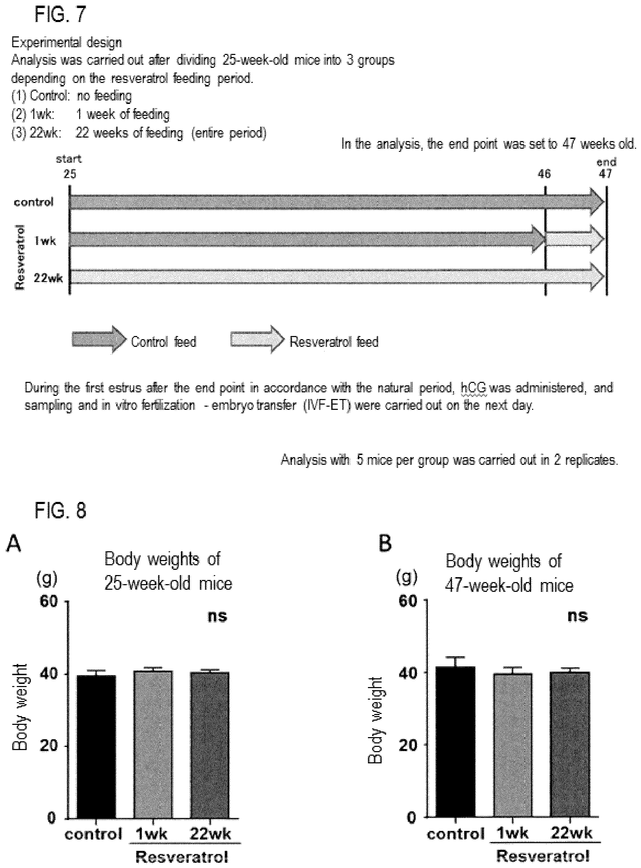

Fifteen female ICR mice of 25 weeks old (CLEA Japan Inc., Tokyo, Japan) were randomly divided into 3 cages at 5 mice per cage. The mice were kept at a room temperature of 22°C at a humidity of 55% in a 12-hour light/dark cycle. Each mouse was fed with 6 g per day of mouse feed, which was MF control feed (Oriental Yeast Co., Ltd., Tokyo, Japan) or resveratrol (3,4',5-trihydroxy-trans-stilbene (trans-1,2-(3,4',5-trihydroxydiphenyl)ethylene), Tokyo Chemical Industry Co., Ltd., Tokyo, Japan)-containing feed (containing 0.04% by mass resveratrol), in the schedule shown in the experimental design in FIG. 7. The mice were provided with water ad libitum. During the feeding period, the body weight was measured once a week. The body weights (group average) of each group of mice at Week 25 and the body weights (group average) of each group of mice at Week 47 are shown in FIG. 8.

Handling and rearing of all mice were carried out according to a guideline provided by the laboratory animal facility of St. Marianna University School of Medicine.

As shown in FIG. 8, at the beginning of the test, that is, among the 25-week-old mice, no significant difference in the body weight was found between the control group and the group in which resveratrol is to be administered for 22 weeks. Also at the end of the test, that is, among the 47-week-old mice, no significant difference in the body weight was found between the control group and the group in which resveratrol was administered for 22 weeks.

### (In Vitro Fertilization and Embryo Culture)

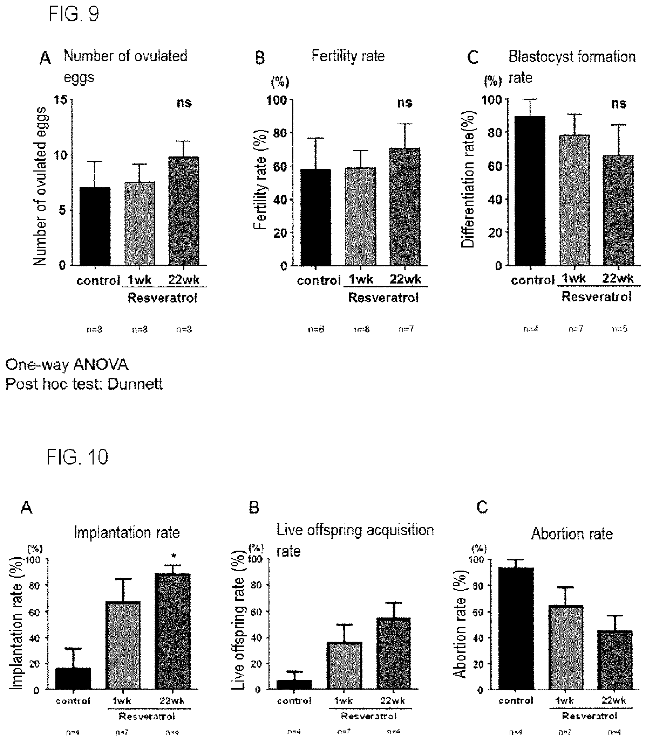

According to the method described in Example 1, mice that became older than 47 weeks old in each group were subjected to in vitro fertilization and embryo culture. The number of ovulated eggs, the fertility rate, and the blastocyst formation rate were calculated. The results are shown in FIG. 9.

As shown in FIG. 9, no significant difference in the number of ovulated eggs, the fertility rate, or the blastocyst formation rate was found between the control group and the group in which resveratrol was administered for 22 weeks.

### (Embryo Transfer and Cesarean Section)

According to the method in Example 1, embryos that reached the blastocyst stage were subjected to embryo transfer and cesarean section. The implantation rate, the live offspring acquisition rate, and the abortion rate were calculated. The results are shown in FIG. 10.

As shown in FIG. 10, a significant difference in the implantation rate was found between the control group and the group in which resveratrol was administered for 22 weeks (P<0.05; One-way ANOVA, Post hoc test: Dunnett's method). As a result of the administration of resveratrol for 22 weeks, an increase in the implantation rate was found. On the other hand, no statistically significant difference was found in the live offspring acquisition rate or the abortion rate. However, the live offspring acquisition rate tended to increase, and the abortion rate tended to decrease.

Pterostilbene was shown to be more effective for, for example, increasing the number of ovulated eggs, increasing the live offspring rate, and decreasing the abortion rate compared to the stilbene derivative resveratrol. It was thus shown that pterostilbene has an excellent action for prevention or improvement/treatment of deterioration of ovarian function.

### <Example 2>

### (Experimental Animals)

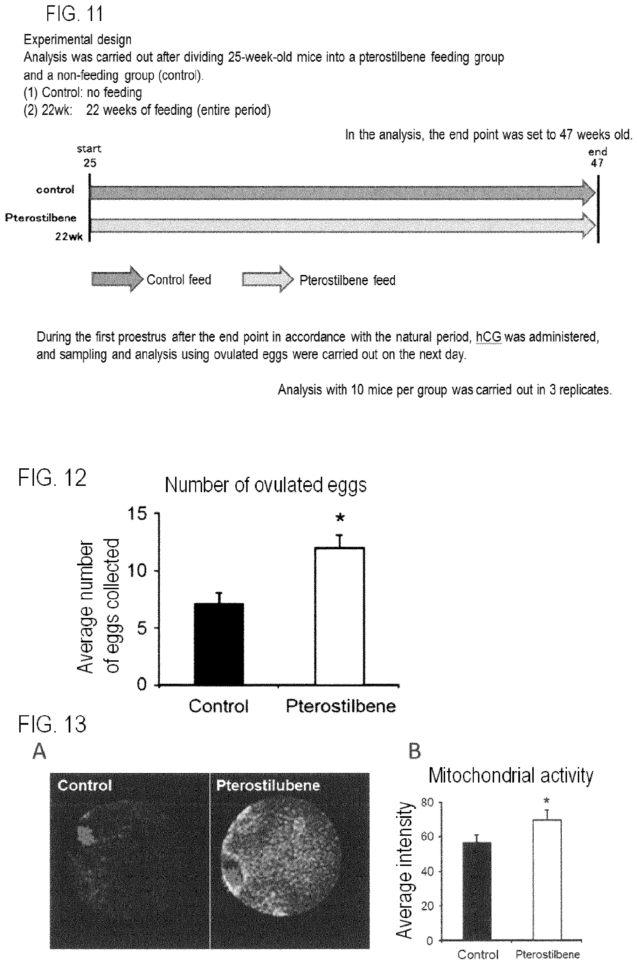

Twenty female ICR mice of 25 weeks old (CLEA Japan Inc., Tokyo, Japan) were randomly divided into 4 cages at 5 mice per cage. The mice were kept at a room temperature of 22°C at a humidity of 55% in a 12-hour light/dark cycle. Each mouse was fed with 6 g per day of mouse feed, which was MF control feed (Oriental Yeast Co., Ltd., Tokyo, Japan) or pterostilbene (90% Silvinol, Sabinsa Japan Corporation, Tokyo, Japan)-containing feed (containing 0.04% by mass pterostilbene), in the schedule shown in the experimental design in FIG. 11. The mice were provided with water ad libitum. This test was carried out in three replicates, and a total of 30 mice per group were used for the experiment.

Handling and rearing of all mice were carried out according to a guideline provided by the laboratory animal facility of St. Marianna University School of Medicine.

### (Collection of Ovulated Eggs)

For mice that became older than 47 weeks old, the sexual cycle was investigated by the vaginal smear test. During the proestrus, 10 IU of Gonadotropin (ASKA Pharmaceutical Co., Tokyo, Japan) was intraperitoneally administered. Fifteen hours after the administration, cumulus oocyte complexes (COCs) were collected from oviduct. Cumulus cells were then detached using M16 medium + hyaluronidase to isolate eggs. In this process, the ovulation number in each group was recorded. The results are shown in FIG. 12.

As shown in FIG. 12, a significant difference in the number of ovulated eggs was found between the control group and the pterostilbene administration group (P<0.05; t-test). As a result of the administration of pterostilbene for 22 weeks, an effective increase in the number of ovulated eggs was found.

### (Analysis of Mitochondrial Activity in Eggs)

The isolated eggs were treated for 10 minutes with M16 medium supplemented with Mitotracker and Hoechst 33342. Thereafter, washing was carried out three times with M16 medium, and observation and measurement of the fluorescence intensity were carried out using a confocal laser microscope (LSM 510: Zeiss). The results are shown in FIG. 13.

As shown in FIG. 13, a significant difference in the mitochondrial activity in the eggs was found between the control group and the pterostilbene administration group (P<0.05; t-test). As a result of the administration of pterostilbene for 22 weeks, effective enhancement of the mitochondrial activity in the eggs was found.

### (Measurement of ATP Amount in Eggs)

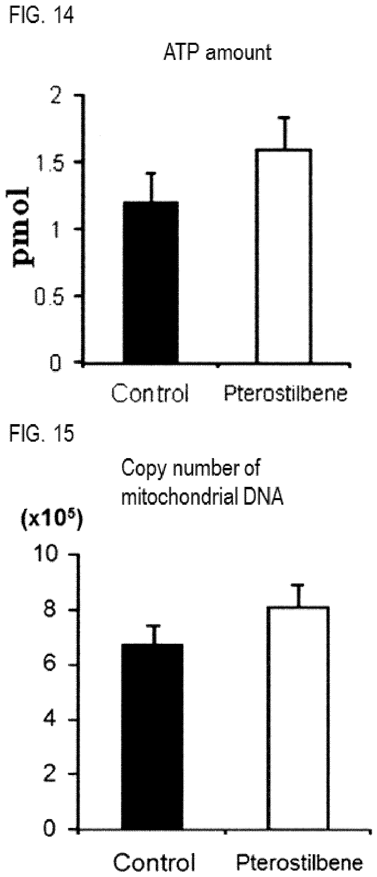

The isolated eggs were treated with acidified Tyrode's solution to remove the zona pellucida. The obtained ooplasm was transferred to a 1.5-mL microtube together with 5 µL of M16 medium, and stored at -20°C. On another day, the ATP amount was measured by measuring luminescence using an ATP-Glo assay kit with a multispectro-microplate reader (Varioskan Flash: Thermo Scientific). The results are shown in FIG. 14.

As shown in FIG. 14, no statistically significant difference in the ATP content in the eggs was found between the control group and the pterostilbene administration group. However, the pterostilbene administration group tended to show a higher ATP content compared to the control group.

### (Analysis of Copy Number of Mitochondrial DNA in Eggs)

The isolated eggs were treated with acidified Tyrode's solution to remove the zona pellucida. The obtained ooplasm was transferred to a 1.5-mL microtube together with 10 µL of lysis buffer, and stored at -20°C. On another day, real-time PCR was carried out using the resulting cell lysate as a template. From the result obtained, the copy number of mitochondrial DNA was calculated. The results are shown in FIG. 15.

No statistically significant difference in the copy number of mitochondrial DNA or the ATP content in the eggs was found between the control group and the pterostilbene administration group. As a result of the administration of pterostilbene for 22 weeks, no effective increase of the copy number of mitochondrial DNA in the eggs was found.

### <Example 3>

### (Measurement of Blood Pterostilbene and Resveratrol Concentrations)

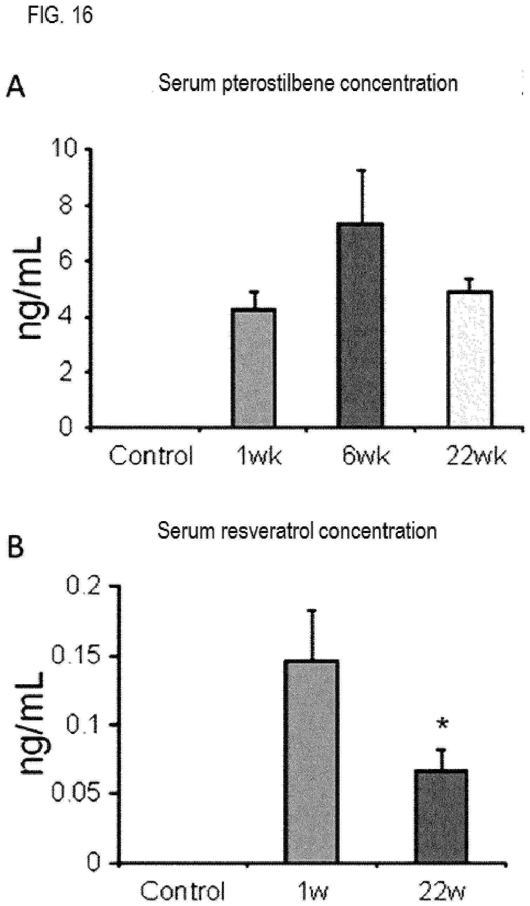

Serum samples recovered when the fertility tests in Example 1 and Comparative Example 1 were carried out, which samples had been stored at -80°C, were used to perform the measurement. The serum samples were sent to Sumika Chemical Analysis Service, Ltd. while being kept in a frozen state. LC-MS and HPLC analyses were requested. The results are shown in FIG. 16.

As shown in FIG. 16, pterostilbene could be detected in the serum in the groups in which pterostilbene was administered. In contrast, pterostilbene could not be detected at all in the non-administration group (control). Similarly, resveratrol could be detected only in the administration groups. In the pterostilbene test groups, there was no significant difference in the concentration in the blood among the administration groups. It was thus thought that pterostilbene had been stably present in blood irrespective of the dosing period. In contrast, in the resveratrol test groups, the concentration in the blood in the long-term administration group (22 wk) was significantly lower than that in the short-term administration group (1 wk) (P<0.05; t-test).

### <Reference Example>

### (Trans-resveratrol Administration Experiment)

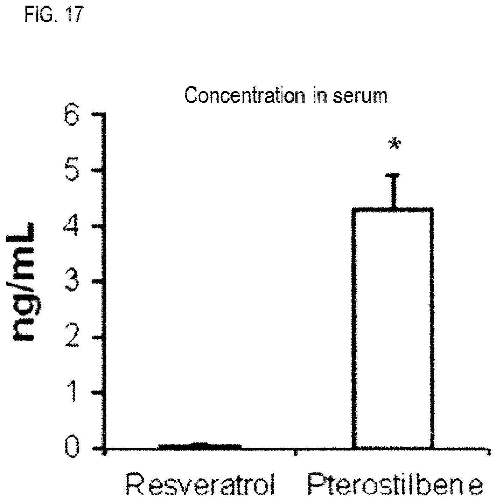

Pterostilbene is a methylated structure of resveratrol, and more stable than trans-resveratrol, which is commonly used. By completely the same design as the experimental design of the addition experiment for pterostilbene administration in Example 2, a trans-resveratrol addition administration test was carried out, and the concentration in the blood was compared to study stability in the body. The results are shown in FIG. 17.

As shown in FIG. 17, the concentration in the serum in the pterostilbene administration group was about 70 times higher than the concentration in the serum in the trans-resveratrol administration group (P<0.05; t-test). Although the results cannot be generalized since these substances are not completely the same, pterostilbene was found to be more stably present in blood under the same administration conditions, so that usefulness of pterostilbene was demonstrated.

### <Example 4>

### (Analysis of Ovarian Tissue)

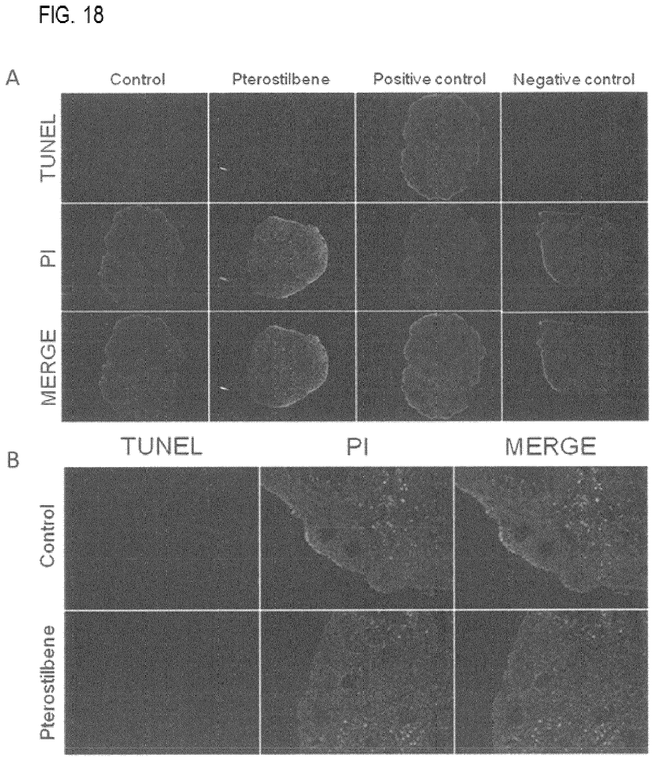

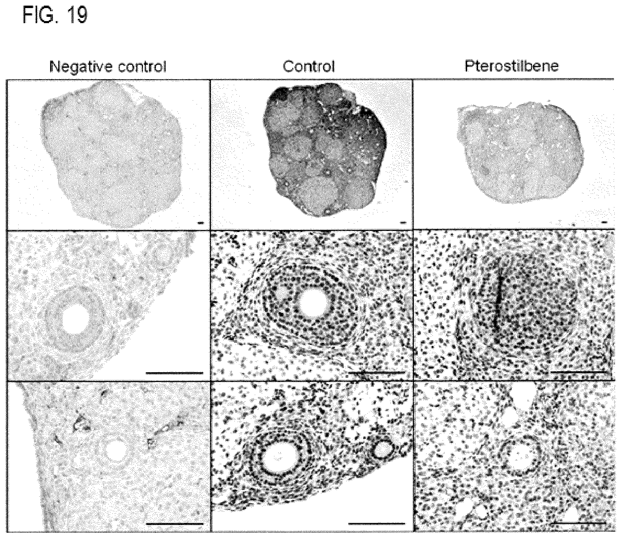

Ovarian tissues (control and 22 wk) recovered in the fertility test in Example 1, which had been stored after formalin fixation, were used to carry out tissue analysis. The ovarian tissues were embedded in paraffin, and sections with a thickness of 4 µm were prepared therefrom using a microtome. Using the sections prepared, TUNEL staining and 8-OHdG staining were carried out, and the sections were observed using a fluorescence microscope (BZ-X710: KEYENCE). In the TUNEL staining, apoptosis of cells was detected, and, in the 8-OHdG staining, oxidative damage of cellular DNA was studied. The results are shown in FIGs. 18 and 19.

As shown in FIGs. 18 and 19, possible decreases in apoptosis and oxidative damage were shown in the ovary of the pterostilbene administration group compared to the ovary of the control, that is, the non-administration group.

### INDUSTRIAL APPLICABILITY

The present invention is applicable to pharmaceuticals, food or beverage, and the like.

## Claims

1. Pterostilbene for use in preventing or improving deterioration of ovarian function,
wherein the reduction of an implantation rate of fertilized eggs is suppressed, or the implantation rate of fertilized eggs is increased;
wherein the reduction of a live offspring rate is suppressed, or the live offspring rate is increased;
wherein the abortion rate is reduced; or
wherein the deterioration of ability of reproduction is improved.

2. An agent for use in preventing or improving deterioration of ovarian function, wherein the agent comprises pterostilbene,
wherein the reduction of an implantation rate of fertilized eggs is suppressed, or the implantation rate of fertilized eggs is increased;
wherein the reduction of a live offspring rate is suppressed, or the live offspring rate is increased;
wherein the abortion rate is reduced; or
wherein the deterioration of ability of reproduction is improved.

3. A pharmaceutical composition for use in preventing or improving deterioration of ovarian function, the composition comprising pterostilbene,
wherein the reduction of an implantation rate of fertilized eggs is suppressed, or the implantation rate of fertilized eggs is increased;
wherein the reduction of a live offspring rate is suppressed, or the live offspring rate is increased;
wherein the abortion rate is reduced; or
wherein the deterioration of ability of reproduction is improved.

4. A food or beverage composition for use in preventing or improving deterioration of ovarian function, the composition comprising pterostilbene,
wherein the reduction of an implantation rate of fertilized eggs is suppressed, or the implantation rate of fertilized eggs is increased;
wherein the reduction of a live offspring rate is suppressed, or the live offspring rate is increased;
wherein the abortion rate is reduced; or
wherein the deterioration of ability of reproduction is improved.

5. The pterostilbene for use according to claim 1, the agent for use according to claim 2, the pharmaceutical composition for use according to claim 3, the food or beverage composition for use according to claim 4, wherein the subject is a human.

6. The pterostilbene for use according to claim 5, the agent for use according to claim 5, the pharmaceutical composition for use according to claim 5, the food or beverage composition for use according to claim 5, wherein the subject has deterioration of ovarian function caused by aging.

7. The pterostilbene for use according to claim 5, the agent for use according to claim 5, the pharmaceutical composition for use according to claim 5, the food or beverage composition for use according to claim 5, wherein the subject has infertility.

## Patentansprüche

1. Pterostilben zur Verwendung zum Verhindern oder Verbessern einer Beeinträchtigung der Eierstockfunktion,
wobei die Verringerung der Einnistungsrate befruchteter Eizellen unterdrückt bzw. die Einnistungsrate befruchteter Eizellen erhöht wird;
wobei die Verringerung der lebender Nachkommenrate unterdrückt bzw. die lebender Nachkommenrate erhöht wird;
wobei die Fehlgeburtrate reduziert wird oder
wobei die Beeinträchtigung der Fortpflanzungsfähigkeit verbessert wird.

2. Wirkstoff zur Verwendung zum Verhindern oder Verbessern einer Beeinträchtigung der Eierstockfunktion, wobei der Wirkstoff Pterostilben umfasst,
wobei die Verringerung der Einnistungsrate befruchteter Eizellen unterdrückt bzw. die Einnistungsrate befruchteter Eizellen erhöht wird;
wobei die Verringerung der lebender Nachkommenrate unterdrückt bzw. die lebender Nachkommenrate erhöht wird;
wobei die Fehlgeburtrate reduziert wird oder
wobei die Beeinträchtigung der Fortpflanzungsfähigkeit verbessert wird.

3. Pharmazeutische Zusammensetzung zur Verwendung zum Verhindern oder Verbessern einer Beeinträchtigung der Eierstockfunktion, wobei die Zusammensetzung Pterostilben umfasst,
wobei die Verringerung der Einnistungsrate befruchteter Eizellen unterdrückt bzw. die Einnistungsrate befruchteter Eizellen erhöht wird;
wobei die Verringerung der lebender Nachkommenrate unterdrückt bzw. die lebender Nachkommenrate erhöht wird;
wobei die Fehlgeburtrate reduziert wird oder
wobei die Beeinträchtigung der Fortpflanzungsfähigkeit verbessert wird.

4. Lebensmittel- oder Getränkezusammensetzung zur Verwendung zum Verhindern oder Verbessern einer Beeinträchtigung der Eierstockfunktion, wobei die Zusammensetzung Pterostilben umfasst,
wobei die Verringerung der Einnistungsrate befruchteter Eizellen unterdrückt bzw. die Einnistungsrate befruchteter Eizellen erhöht wird;
wobei die Verringerung der lebender Nachkommenrate unterdrückt bzw. die lebender Nachkommenrate erhöht wird;
wobei die Fehlgeburtrate reduziert wird oder
wobei die Beeinträchtigung der Fortpflanzungsfähigkeit verbessert wird.

5. Pterostilben zur Verwendung nach Anspruch 1, Wirkstoff zur Verwendung nach Anspruch 2, pharmazeutische Zusammensetzung zur Verwendung nach Anspruch 3, Lebensmittel- oder Getränkezusammensetzung zur Verwendung nach Anspruch 4, wobei es sich bei dem Individuum um einen Menschen handelt.

6. Pterostilben zur Verwendung nach Anspruch 5, Wirkstoff zur Verwendung nach Anspruch 5, pharmazeutische Zusammensetzung zur Verwendung nach Anspruch 5, Lebensmittel- oder Getränkezusammensetzung zur Verwendung nach Anspruch 5, wobei das Individuum eine altersbedingte Beeinträchtigung der Eierstockfunktion aufweist.

7. Pterostilben zur Verwendung nach Anspruch 5, Wirkstoff zur Verwendung nach Anspruch 5, pharmazeutische Zusammensetzung zur Verwendung nach Anspruch 5, Lebensmittel- oder Getränkezusammensetzung zur Verwendung nach Anspruch 5, wobei das Individuum unfruchtbar ist.

## Revendications

1. Ptérostilbène pour une utilisation dans la prévention ou l'amélioration d'une détérioration d'une fonction ovarienne,
la réduction d'une vitesse d'implantation d'œufs fertilisés étant supprimée, ou la vitesse d'implantation d'œufs fertilisés étant augmentée ; la réduction d'un taux de descendants vivants étant supprimée, ou le taux de descendants vivants étant augmenté ;
le taux d'avortement étant réduit ; ou
la détérioration de la capacité de reproduction étant améliorée.

2. Agent pour une utilisation dans la prévention ou l'amélioration de la détérioration d'une fonction ovarienne, l'agent comprenant du ptérostilbène,
la réduction d'une vitesse d'implantation d'œufs fertilisés étant supprimée, ou la vitesse d'implantation d'œufs fertilisés étant augmentée ; la réduction d'un taux de descendants vivants étant supprimée, ou le taux de descendants vivants étant augmenté ;
le taux d'avortement étant réduit ; ou
la détérioration de la capacité de reproduction étant améliorée.

3. Composition pharmaceutique pour une utilisation dans la prévention ou l'amélioration de la détérioration d'une fonction ovarienne, la composition comprenant du ptérostilbène,
la réduction d'une vitesse d'implantation d'œufs fertilisés étant supprimée, ou la vitesse d'implantation d'œufs fertilisés étant augmentée ; la réduction d'un taux de descendants vivants étant supprimée, ou le taux de descendants vivants étant augmenté ;
le taux d'avortement étant réduit ; ou
la détérioration de la capacité de reproduction étant améliorée.

4. Composition alimentaire ou de boisson pour une utilisation dans la prévention ou l'amélioration de la détérioration d'une fonction ovarienne, la composition comprenant du ptérostilbène,
la réduction d'une vitesse d'implantation d'œufs fertilisés étant supprimée, ou la vitesse d'implantation d'œufs fertilisés étant augmentée ; la réduction d'un taux de descendants vivants étant supprimée, ou le taux de descendants vivants étant augmenté ;
le taux d'avortement étant réduit ; ou
la détérioration de la capacité de reproduction étant améliorée.

5. Ptérostilbène pour une utilisation selon la revendication 1, agent pour une utilisation selon la revendication 2, composition pharmaceutique pour une utilisation selon la revendication 3, composition alimentaire ou de boisson pour une utilisation selon la revendication 4, le sujet étant un humain.

6. Ptérostilbène pour une utilisation selon la revendication 5, agent pour une utilisation selon la revendication 5, composition pharmaceutique pour une utilisation selon la revendication 5, composition alimentaire ou de boisson pour une utilisation selon la revendication 5, le sujet ayant une détérioration d'une fonction ovarienne causée par le vieillissement.

7. Ptérostilbène pour une utilisation selon la revendication 5, agent pour une utilisation selon la revendication 5, composition pharmaceutique pour une utilisation selon la revendication 5, composition alimentaire ou de boisson pour une utilisation selon la revendication 5, le sujet présentant une infertilité.
